# EUROPEAN PATENT SPECIFICATION

(11) **EP 1 503 776 B1**
(45) Date of publication and mention of the grant of the patent: **21.12.2011**
(21) Application number: 03733913.2
(22) Date of filing: 01.05.2003
(51) Int. Cl.: A61K 35/74, A61K 36/06, C12N 1/00, C12N 1/20, C12N 1/14, C12N 1/16, C12N 1/18

(54) **METHOD OF ENHANCING BIOLOGICAL ACTIVITY OF PLANT EXTRACTS**
VERFAHREN ZUR VERBESSERUNG DER BIOLOGISCHEN AKTIVITÄT VON PFLANZENEXTRAKTEN
TECHNIQUE PERMETTANT DE RENFORCER L'ACTIVITE BIOLOGIQUE D'EXTRAITS DE PLANTE

(30) Priority: 02.05.2002 US 377582 P
(43) Date of publication of application: 09.02.2005
(73) Proprietor: E-L Management Corp., New York, NY 10153 (US)
(72) Inventor: CIOCA, George, Lake Grove, NY 11755 (US); IONITA-MANZATU, Vasile, Old Bethpage, NY 11804 (US); GEORGE, Liliana, Centerport, NY 11721 (US); GIACOMONI, Paolo, Commack, NY 11725 (US); MAMMONE, Thomas, Farmingdale, NY 11735 (US); COLLINS, Donald, F., Plainview, NY 11803 (US); MAES, Daniel, H, Plainview, NY 11743 (US); BEVACQUA, Andrew, J., East Setauket, NY 11723 (US); GEDEON, Harvey, Tenafly, NY 07670 (US); FTHENAKIS, Christina, Dix Hills, NY 11746 (US); TADLOCK, Charles, C., Islip Terrace, NY 11752 (US)
(74) Representative: Hirsch & Associés
(86) International application number: PCT/US2003/013473
(87) International publication number: WO 2003/092711

(56) References cited:
- EP-A- 1 279 729
- US-A- 4 301 251
- US-A- 4 301 251
- US-A- 5 466 452
- US-A- 5 466 452
- US-A- 5 846 397
- US-A- 5 846 397
- US-A- 6 139 855
- US-A- 6 139 855

## Description

### Field of the Invention

The invention relates to cosmetic and pharmaceutical compositions, and methods of making same. More specifically, the invention relates to methods of making cosmetic and pharmaceutical compositions with improved biological activity.

### Background of the Invention

In recent years, consumers of skin care products have become increasingly aware of the contents of the products they use. The demand for products based on "natural" materials has increased significantly. Plants have, for thousands of years, been the source of numerous folk remedies as well as providing the basis for the development of pharmaceuticals, for example, the best painkiller, aspirin, is a modification of the weeping-willow-derived salicylic acid, and the cancer treatment, Taxol, was ultimately derived from yews (genus *Taxus*). A renewed interest in using plant materials for the treatment of skin has arisen in connection with the overall desire to return to a simpler or more natural way of life, and to avoid human- or animal-derived materials which might be contaminated by unwanted material or considered undesirable by some consumers or government agencies.

The use of plant materials is not without its difficulty, however. Frequently, a plant may not produce large quantities of the compound of interest, making it difficult to obtain meaningful quantities for commercial therapeutic purposes. In order to maintain adequate supplies of a material, it may be necessary to gather large volumes of the plant material in question, which in turn can lead to environmental damage, ecological disruption, or in the worst case, ultimate extinction of the plant. When the identity of the compound exhibiting the desired activity is known, it is sometimes possible to make the compound synthetically, i.e., completely by chemical pathways, or to create the desired compound semi-synthetically, by starting with a more abundant phytochemical precursor and depriving the desired compound by chemical pathways. Either of these procedures can be costly, however, and in some individuals' perceptions, can make the final product somehow less "natural". Indeed, in many cases, the natural product will be perceived as being far superior to its synthetic counterpart, notwithstanding their presumed chemical identity; for instance, in the case of an asymmetric molecule, the naturally-derived molecule will ordinarily have one chirality, whereas synthetic chemicals will have all the possible chiralities. A very good example of the importance of natural origin is the nearly universal perception of the superiority of natural vanilla compared with artificial vanilla.

In more recent times, it has also been possible to obtain larger quantities of desired phytochemicals by plant cell culture which selects for cells producing larger quantities of the compound of interest, or genetic transformation of easily proliferated host cells, such as bacteria, with plant cell genes to allow production of a plant material by the host. However, there is also a segment of the population that objects, on environmental or theological bases, to these means for obtaining desirable chemicals.

There thus clearly exists a current need for a means to enhance the availability of biologically active plant-derived compounds, in a way that maintains the natural character of the product as a whole. The present invention now provides a means for increasing the biological activity of plant extracts, thereby effectively increasing the availability of the compound of interest.

### Summary of the Invention

The present invention relates to a method, herein called bioconversion of modulating a selected biological activity of a naturally occurring material having one or more biological activities or biochemical properties in a suitable extract of the natural material. The method of the invention comprises the incubating the extracts in the presence of a yeast or other suitable micro-organisms, under suitable aerobic conditions, for a period of time sufficient to increase or otherwise alter favorably the selected activity. Preferably the activity is increased at least 100% over baseline activity of the untreated extract. The invention also relates to the modified extract made by this process.

### Detailed Description of the Invention

It has now unexpectedly been discovered that it is possible to enhance or modulate the activity of a naturally occurring material, for example, a plant extract already possessing biological activity, by relatively simple processing of the extract in the presence of yeast. The process of bioconversion, as it is referred to herein, is aerobic, and is reminiscent of the type of process that converts wine to vinegar.

The basic process is as follows: a naturally occurring material is selected that has one or more biological activities, or a certain biochemical properties such as solubility or aroma. For the sake of simplicity, throughout the specification and claims, "biological activity" will be understood to encompass both true biological activity as well as biochemical properties. In this regard, it should also be understood that, while in many cases, the desired effect is to enhance the natural activity by increasing its potency, in other cases, particularly when modifying a biochemical property, the property is not so much enhanced as modulated, in that the property is improved qualitatively, but not necessarily quantitatively. Thus, throughout the specification and claims, the term "modulate" or "modulating" is intended to encompass not only an increase in a biological activity, but a qualitative change in a biochemical property. It is not essential to know the chemical identity of the compound responsible for the activities or biochemical properties. An extract of the natural material can be made by incubating the material with distilled water, aqueous solutions of salts, structured waters, water/alcohol mixtures, or biologically acceptable oil, for a period of time sufficient to extract active materials from the material. This time period may be as short as several hours, to as long as a week or so. If the identity of the molecule is known, then analysis of the presence of the molecule in the extraction fluid will indicate adequate extraction; however, if the identity is not known, observation of change of color of the solvent, or simply leaving the extract for up to a week, is an alternate means for ensuring reasonably adequate extraction. As an alternative to using a crude extract, the starting substrate can be a substantially pure ingredient, for example, a commercially available plant extract or purified plant component or pure synthetic compound having biological activity. For convenience, all these types of starting material shall be referred to herein as an "extract", and such materials will also be referred to as "plant-derived" even if ultimately prepared synthetically. The extract is then combined with yeast or other suitable microorganism at room temperature, under well-aerated conditions, e.g. stirred at 20-200 rpm with bubbling air at 0.2-2 liters /minute, for a period of at least about 24 hours, preferably longer.

The bioconversion process can take either one of two approaches. The first is a process in which the microorganism is incubated not only with the extract but also with traditional culturing nutrients. During such an incubation, the yeast can multiply. The second, and preferred, approach, is to incubate the microorganism in an aqueous environment, in the presence of only the extract, and in the substantial absence of any additional nutrients. During this method of processing, the yeast do not multiply, but engage in the catabolic processing of the starting material. The bioconversion is monitored periodically for signs of the plateauing of biological activity, for example, a leveling off of pH, and then the system temperature is raised to between about 30-50°C, preferable about 40-45°C, for at least about 24 hours. In one embodiment, the temperature is then briefly raised to 90-95°C for a period of about 5-10 minutes, which ruptures the yeast, releasing the cell contents. Alternately, the cells can be disrupted by sonication. The entire system is then cooled to room temperature, and filtered with progressively decreasing pore size to remove yeast debris, leaving an extract that has an enhanced level of activity or modified biochemical properties in comparison with the unprocessed extract.

Using such procedures, the level of relevant activity is very significantly increased, preferably increased at least about 25%, more preferably at least about 50%, most preferably at least about 100%, and often is increased significantly more, i.e., three- to tenfold. The organism used for the biotechnological treatment, or bioconversion, of the extract can be any microorganism that is normally used for this purpose. A particularly useful organism is a standard brewer's yeast, *Saccharomyces cerevisiae*, but other aerobic microorganisms, including but not limited to *Aspergillus nidulans, Saccharomyces pombe, Thermus aquaticus, Bacillus subtilis,* cyanobacteria, or archaebacteria can also be used.

The concentration of microorganism used in the conversion process is not critical, and may be relatively small, i.e., from about 0.01% to about 1% by weight of the mixture, particularly in the embodiment in which the yeast do not multiply during conversion. Greater amounts than this can also be used. The amount of starting extract is also not critical; however, if it is desired to prevent yeast proliferation, the amount should be controlled so as not to provide enough nutrient to the yeast so to allow multiplication. Ordinarily, the amount will be about 0.01 to about 10%, preferably about 0.01 to about 5%, of active material, the concentration depending on the starting material as well as on its solubility.

In one embodiment of the invention as described above, the fluid medium in which the active material is extracted and/or in which the bioconversion takes place is simply water. In a preferred embodiment, the water used, however, is a structured water, i.e., I water, S water, or a combination of the two, as described, for example, in RO 88053 [S-type water], and RO 88054 (I-type water], and US Patent Nos. 5,846,397 and 6139855. The use of structured water in one or both of these phases of the bioconversion process can further enhance the sought-after properties of the active material, and in some cases can make the difference between a successful and unsuccessful bioconversion As a general rule, when the clustering in structure water(s) enhances the biological properties or modifies the biochemical behavior of a particular material in the absence of bioconversion, as is described in 5,846, 397 and 6,139, 855, then conducting the extraction and/or bioconversion process in the presence of structured water(s) can also improve the results. As also noted above, the bioconversion medium is generally not supplied with nutrients sufficient to support the growth and multiplication of the bioconverting organism, so that the sole source of substrate for the organism's biochemical activity is the active material provided. However, in an alternate embodiment, successful bioconversion can be performed in the presence of a nutrient medium appropriate to the growth of the microorganism.

The natural material selected for bioconversion can be any natural material having a biological activity or a biochemical property which it would be desirable to improve. The initial material from which the extract is made can be a relatively crude extract of any plant, or plant part, known to have some level of biological activity or property, whether or not the chemical identity of the active component or components is known. A wide variety of plants containing active materials are well-known and documented. Examples of such can be found in D'Amelio, F.S., Sr., Botanicals, a Phytocosmetic Desk Reference, CRC Press, 1999, and Bruneton, J., Pharmacognosy, Phytochemistry, Medicinal Plants, Lavoisier Publishing, 1995. Some specific examples of such plants include, licorice (*Glycyrrhiza*) extract, as an agonist for estrogen receptors, an inhibitor of tyrosinase (skin whitening), and a mitochondrial protector; ferulic acid or its derivatives, as a whitening agent; rosemary extract, as an inducer of p450, an inhibitor of mast cell degranulation, and an antioxidant; chamomile (*Matricaria*) for whitening, and in combination with spinach extracts, as enhancers of gap- junctional communication, inhibition of cyclooxygenase and iNOS, and inhibition of mast cell degranulation; lavender, for inhibition of histamine release and in combination with rosemary for P450 induction, enhancing activity of glutathione-S-transferase and inhibition of mast cell degranulation; ginger (*Zingiber*), for its digestive and anti-inflammatory properties, and in combination with rose extract for modulation of estrogen receptors; juniper and spinach combined, for inhibition of prostaglandin synthesis; white or green leaf teas, as antioxidants, white birch, as a protease inhibitor and inducer of heat shock proteins, grape seed or other grape extracts, as antioxidants; *Pterocarpus ulei,* as antioxidant, inhibitor of hyaluronidase, and anti-inflammatory; *Centella asiatica,* for collagen stimulation; *Aniba purchyriminor,* as an antioxidant, an inhibitor of platelet activating factor and as an anti-angiogenic agent; *Echinacea,* for its wound-healing and immunity-boosting properties; *Aloe vera,* for anti-irritant properties, and anti-adhesion; St. John's wort (*Hypericum*) for anti-depressant and astringent properties; neem (*Azadirachta*) as insect repellent; and *Mimosa pudica*, for collagenase inhibition properties. These are just a few examples, and others will be readily apparent to those skilled in the art.

The starting material can also be animal-derived material, such as chitin, keratin, cartilage, collagen, and the like. Alternatively, the starting material may be a substantially pure plant- or animal-derived or chemically synthesized compound known to have biological activity, for example, any number of flavonoids, isoflavonoids, or anthocyanins that are known to have biological activities; amino acids and derivatives thereof, such as N-acetyl cysteine and N-acetyl glutamine, aldosamines, which term includes derivatives thereof, such as N-acetyl glucosamine, xanthines, which term includes derivatives thereof, such as caffeine, antioxidants, such as resveratrol, or rosmarinic acid, antiinflammatory compounds, such as glycyrrhizin, glycyrrhetinic acid, and related compounds; or steroids and steroid precursors, such as DHEA or natural precursors thereof.. It will also be understood that many of these compounds have more than one biological activity; for example, resveratrol is both an antioxidant and a whitening agent. As used herein, the use of the term "natural materials" is intended to encompass not only material derived directly from nature, but also materials that are present in natural sources, but which may have been made by synthetic or semi-synthetic means.

The materials described herein are primarily materials having biological activity that is useful for topical application to enhance therapeutic or beneficial effects of the materials on the skin, but those skilled in the art will readily recognize that to the extent the function enhanced is applicable to other organ systems, the improved materials can be used systemically as well. It will also be understood that this method can be readily applied to naturally occurring material of any kind that has biological activity applicable solely or primarily to systemic use as well. Examples of herbal and other plant materials known to have systemic medicinal effects are described in both D'Amelio and Bruneton, *supra*. Particular examples of materials having interesting biochemical properties are fragrance or essential oils, which can have both cosmetic benefits as well as systemic benefits, particularly in the area of aromatherapy. Essential oils and perfumes have been used for thousands of years, not just for their odor-disguising properties but because of their recognized effects on the psyche as well as the body. A useful reference for this information is found in Groom, N, : Perfume, the ultimate guide to the world's finest fragrances, Running Press, Philadelphia-London, 1999, incorporated herein by reference. Examples of useful essential oils, or plant materials that are known to have such benefits are rose essential oil(decreases tension), rosemary (invigorating), lavender (relaxation), lemon (anti-depressant), neroli (reduces anxiety), clary sage (antidepressant), basil (increases alertness and memory), aniseed (carminative), melissa (anti-depressant), sandalwood (mood-elevating), peppermint (mental stimulant), or ylang-ylang (increases sensuality). Each of these materials, if subjected to bioconversion, can benefit by having these individual properties enhanced.

A particularly useful observation is that the process of bioconversion may succeed in rendering an essential oil water soluble, either by modifying its chemical structure or by complexing it with the hydrophobic domains of water-soluble macromolecules, which are exposed to the extract during the step of pyrolysis. Geraniol from rose oil, and essential oils from rosemary and lavender have been made water soluble by bio-conversion.

This possibility of rendering hydro-soluble compounds which are normally insoluble in water has fantastic applications to the field of formulation, in which solubility, or lack thereof, of a desirable material in a particular type of vehicle is frequently problematic. Clearly, this observation is therefore not limited to essential oils but encompasses a large variety of compounds used in cosmetics, such as fillers, thickeners, surfactants and actives having difficult solubility in water or oil.

The increase in activity observed with the current bioconversion process is particularly surprising, and has been demonstrated with a wide variety of different naturally occurring materials with a wide range of biological activities, as will be seen from the following examples. While not wishing to be bound by any particular theory, this unexpected occurrence may be due to the solubilization or increased bioavailability of the active molecules, the enzymatic transformation of the actives, the synergistic interaction of the actives with yeast components such as vitamins or nutrients or oligoelements, i.e., those elements that are found in the body at very low concentrations, or a combination of all these processes. Whatever the reason, the successful increase of activity of a broad variety of both crude and relatively pure natural materials, having diverse chemical identities and biological activities, unequivocally shows the broad applicability of the bioconversion technique in enhancing naturally occurring biological activity. The benefit is in the production of a material which is more active than the original at equivalent or even lower concentrations, which in turn provides greater availability of otherwise possibly scarce and/or costly natural materials with significant biological benefits.

The invention is further illustrated by the following examples.

### EXAMPLES

### Example 1

This example illustrates the general process of bioconversion that can be used with a variety of natural materials.

### A. Inoculum Preparation:

Twenty-four hours prior to starting the bioconversion process, a fresh yeast culture of *Saccharomyces cerevisiae*: ATCC 60219 is prepared. A colony from a freshly streaked plate is inoculated into a 400 ml stir flask containing 100% Tryptic Soy Broth. Enough inoculum is made to accommodate for 1% (vol/vol) of the total volume of the bioconversion. This sums up to a ratio yeast/medium of about 0.01% to 0.1% wet weight/volume. Once the volume size of the conversion is determined, a vessel is chosen to accommodate the process.

### B. Preparation of Extract:

The material to extract for bioconversion (e.g. minced plant material) is added to 100 ml of water or a 50:50 water:ethanol mixture, or I water with clustered silver ions at 0.1 ppm or I-water, or S-water or other organic solvents such as butylene glycol at a w/w ratio between 0.01 and 10 %. The plant-solvent mixture is incubated at room temperature for a period of one week. After incubation, the debris remaining is removed by filtration, and, in the case of hydroalcoholic extractions, any alcohol remaining is removed by rotary evaporation, leaving an extract to be added to the bioconversion vessel.

### C. Inoculum:

The vessel contains sterile distilled water or I-water, or S-water or mixtures thereof. To this, the extract is added in proportions varying from 0.01% (as it is often the case for fragrant essential oils) to 10 % (volume/volume). After the addition of the active ingredient or plant extract, the vessel can be inoculated with the *Saccharomyces cerevisiae*, which had been previously prepared as described above. All bioconversion using *S. cerevisiae* are inoculated with 1.0% (of the total volume) of the bioconversion. The stirrer is set to the desired speed, typically between 20-200 rpm, at a temperature of about 25°C, with aeration of 0.2-2 liters/min.

### D. Processing time:

The bioconversion will last from 24 - 72 hours. At 24-hour intervals samples are taken for pH, plate count, odor and visual evaluation, and recorded in a data log. A 20 ml retain is also be taken every 24 hours for stability evaluation. The endpoint of the process is ordinarily determined by monitoring the pH, as an indicator of biological activity, to the point where it stabilizes, and then stopping the conversion process. At this point the temperature is raised to 45°C for one day, then a pyrolytic step, in which the temperature is raised to 90-95°C, is performed for 10-15 minutes.

After pyrolysis, the medium is filtered through filters of decreasing pore size: 8, 2 and 0.22 micrometers. Once through the filter, the sample is preserved with 0.5% phenoxyethanol or other preservatives, or stored at 4 C°, and set aside for further use.

### Example 2

Fermentation products of a number of natural materials are prepared substantially as described in Example 1, or that process modified as indicated, and then tested for to determine their level of activity. The results observed are as follows:

### A. Centella asiatica.

Extracts of *Centella asiatica* are known to have collagen enhancing activity. To attempt to enhance this activity, a sample of 0.1% *Centella asiatica* extract is incubated with 0.1% yeast for 96 hours. The testing is conducted as follows: NHDF cells were seeded and grown to confluence in a 96 well plate prior to being treated. The samples tested were 0.1% dry yeast thermolysed after 10 minutes, 0.1% dry yeast thermolysed after 96 hours, 0.1% (w/v) *Centella asiatica* in dH₂O and 0.1% (w/v) *Centella asiatica* that was subjected to the bioconversion process described above for 96 hours by 0.1% yeast in dH₂O. Each of the samples was sterile filtered and diluted further in media before being tested. The samples were diluted to 10%, 5%, 2.5% and 1.25%. When taking into account that the starting concentration of *Centella* was 0.1% (w/v), the final concentrations of *Centella* tested were 0.00125%, 0.0025%, 0.005% and 0.01 % (w/v). The plate was incubated for 3 days at 37°C / 5% CO₂ before the supernatants were harvested, and stored at -80°C in siliconized tubes until the ELISA was performed. The PIP ELISA (Pan-Vera Technology, Code MK101) was performed as outlined in the protocol supplier by the manufacturer and the results were calculated from the standard curve.

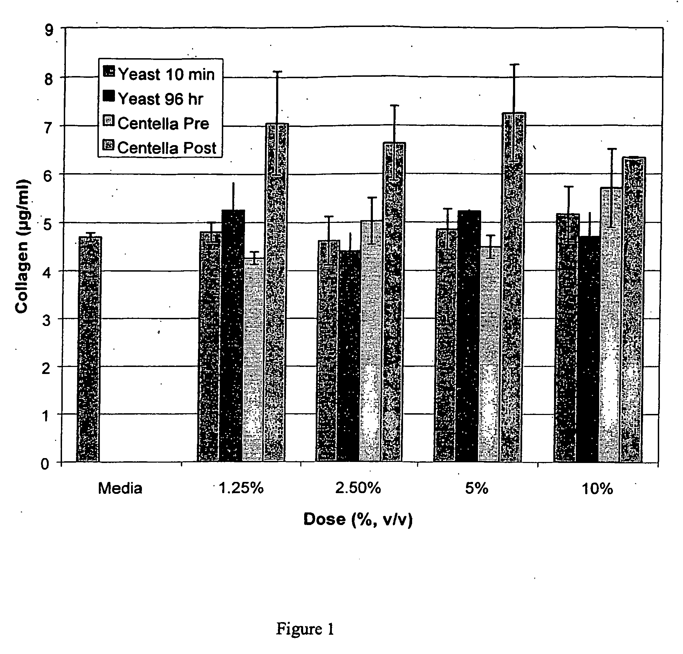

The bioconverted *Ceniella asiatica* sample thermolysed after 96 hours has an *in-vitro* collagen-synthesis stimulating activity which is three to four times larger than the non-bioconverted sample. The yeast control thermolysed after 10 minutes in dH₂O does not induce any change in the synthesis of collagen by cultured human fibroblasts, nor does the yeast sample that was thermolysed 96 hours after being "rehydrated". The results are shown in the graph presented as Figure 1. The results plainly show that an increase in collagen synthesis is provided by the bioconverted *Centella.*

### B. Caffeine

Caffeine in water has substantial antioxidant properties, being capable of reducing the oxidation of lipids by ultraviolet radiation. Caffeine was subjected to bioconversion at concentration as indicated(w/v) in water or in a mixture of structured waters (I/S 60/40). Upon bioconversion in structured water, an increased capability to inhibit the peroxidation of lipids is observed as reported below The methodology for determining inhibition of lipid peroxidation can be found, for example, in Pelle, et al., Ann N.Y. Acad. Sci. 570 : 491-494 (1989).

| Caffeine Concentration | Inhibition of lipid peroxidation | | | |
|---|---|---|---|---|
| | Non bioconverted | | bio-converted | |
| | H2O | I/S water | H2O* | I/S water |
| 0.05% | 30% | 24% | 39%* | 55% |
| 0.25% | 54% | 58% | 53%* | 78% |
| 0.5% | 66% | 66% | 68%* | 87% |

| | | | | |
|---|---|---|---|---|
| * the results in this column indicate a bioconversion conducted in a nutrient containing medium, as described in Example 3 below. | | | | |

### C. Resveratrol

Resveratrol is a well-known antioxidant, able to inhibit the UV induced peroxidation of lipids in a liposomal assay as described by Pelle et al., *supra.* With bioconversion in S-water, its antioxidant properties are boosted, (see table below)

| Concentration of Resveratrol in S water | Anti-oxidant activity Nonbioconverted | Bioconverted |
|---|---|---|
| 0% | 0% | |
| 0.05% | 1.8% | 0.47% |
| 0.25% | 8% | 77% |
| 0.5% | 39% | 92% |

The positive effects of bioconversion on the anti-oxidant properties of resveratrol are not elicited when the process of bioconversion is performed in distilled water.

Resveratrol is also a well-known inhibitor of tyrosinase, the concentration inhibiting 50% of the enzyme activity (IC50) being 0:05%. Bioconversion in S water increases by about 20% the inhibitory potency of resveratrol: after bioconversion in S-water, the IC50 of resveratrol is 0.041 %

### D. White birch

White birch extracts contains compounds which are relatively weak inhibitors of elastase, possibly because of low water solubility. In I/S water (60/40) the IC50 of white birch extract for elastase is of the order of 7.5%. Upon bioconversion in I/S (60/40) waters, its antielastase activity increases by at least ten fold, the IC50 of bioconverted white birch extracts being 0.7%.

### E. Spinach extract

(i)When a culture of fibroblasts is serum starved for 24 hours, DNA synthesis is impaired. An extract of spinach at 0.01-0.1% stimulates DNA synthesis in cultured, serum starved fibroblasts less than twofold, compared to "medium only" negative control, whereas 1% and 10% fetal calf serum restores DNA synthesis, increasing baseline values threefold and sevenfold, respectively. When the spinach extract is bioconverted, the spinach extract at 0.01-.1% increases by a factor of five-seven the synthesis of DNA in serum starved cells and it appears therefore that bioconverted spinach extract behaves like a micronutrient with a DNA-synthesis-stimulatory-activity comparable to the one of 10% serum.
(ii)Serum starved fibroblasts also display damaged morphology, characterized by the presence of numerous vacuoles in the cytoplasm; normal morphology is returned by the addition of 1% or 10% serum. Untreated spinach extract at 0.01-0.1% is unable to restore the healthy morphology, but bioconverted spinach at the same concentrations is capable of restoring the healthy morphology.

### F. Pterocarpus ulei and Aniba purchyriminor

*P. ulei* is an equatorial plant known to have anti-inflammatory properties. *A. purchyriminor* is an equatorial plant containing inhibitors of PAF (Platelet Activating Factor). *A. purchyriminor* is also endowed with a good fragrance. Each one of the plants *Pterocarpus ulei* and *Aniba purchyriminor* has antioxidant activity. The mixture of extracts from the two plants thus constitutes a perfume with helpful biological activities. Their anti-oxidant properties were measured and have observed to be enhanced upon bioconversion. At 0.002%, *Pterocarpus ulei* extracts inhibit UV-induced lipid peroxidation by 64%, but at 0.0005% it has no activity; at 0.002%, *Aniba purchyriminor* inhibits peroxidation by 73%, but at 0.0005%, it inhibits only 23%. The untreated extracts at a concentration 1% each, are combined and diluted to 0.1% each and subjected to bioconversion. The converted extract exhibits a nearly threefold increase of antioxidant activity, in that at 0.0001% it inhibits 12% of the peroxidation, and at 0.0002% it inhibits 26%.

### G. Essential Oils

An essential oil from Rose, composed mainly of geraniol, was made water soluble by bioconversion at a concentration of 0.05%, and tested in water to learn about its effect on mood state. Mood state can be assessed in subjects using the Profile of Mood States (POMS) standard psychological test, which measures tension, depression, anger, vigor, fatigue and confusion. Mood modification can be determined in subjects who fill out the POMS questionnaire before and after sniffing a particular fragrance.

A group of 42 volunteers was requested to fill in a questionnaire before and ten minutes after sniffing bioconverted or non bioconverted rose essential oil, by rating their mood on a scale of 1-11. The outcome of the experiment was that bioconverted rose essential oil decreases tension to a larger extent than non-bioconverted Rose as displayed in the tables below.

Similarly, an essential oil of rosemary was bioconverted at a concentration of 1%. Rosemary essential oil is widely reported in published literature to increase vigor. We found that Bioconverted Rosemary decreases vigor, anger and tension.

**TABLE 1**

| **Material** | **Mood** | **Before** | **After** | **n** | **p** |
|---|---|---|---|---|---|
| | | | | | |
| Biocon Rosemary | Tension | 3.44 | 1.07 | 45 | 0.006 |
| | Anger | 3.24 | 1.78 | 45 | 0.05 |
| | Vigor | | Histogram shift | 45 | |
| Biocon Lavender | Tension | 3.55 | 1.17 | 42 | 0.01 |
| | Vigor | | Histogram shift | 42 | |
| Biocon Lavender + Rosemary | Vigor | | Histogram shift* | 42 | |
| Biocon Rose | Tension | | Histogram shift** | 38 | |

| | | | | | |
|---|---|---|---|---|---|
| *see details in (b) below **see details in (a) below | | | | | |

(a) Mood state number of panelists in the mood state

| | Rose essential oil | | Bioconverted Rose Essential Oil | |
|---|---|---|---|---|
| tension | Before sniffing | after sniffing | Before sniffing | After sniffing |
| 0 | 3 | 3 | 0 | 0 |
| 1 | 3 | 5 | 7 | 9 |
| 2 | 6 | 8 | 8 | 11 |
| 3 | 4 | 5 | 11 | 11 |
| 4 | 2 | 1 | 8 | 3 |
| 5 | 2 | 4 | 0 | 1 |
| 6 | 4 | 2 | 1 | 1 |
| 7 | 3 | 1 | 2 | 1 |
| 8 | 3 | 1 | 1 | 1 |
| 9 | 1 | 3 | 0 | 0 |
| 10 | 1 | 0 | 0 | 0 |
| 11 | 1 | 1 | 0 | 0 |

(b) Mood state number of panelists in the mood state

| | Bioconverted Lavender & Rosemary | |
|---|---|---|
| vigor | Before sniffing | after sniffing |
| 0 | 0 | 0 |
| 1 | 0 | 2 |
| 2 | 4 | 1 |
| 3 | 3 | 11 |
| 4 | 4 | 4 |
| 5 | 10 | 2 |
| 6 | 4 | 4 |
| 7 | 4 | 5 |
| 8 | 5 | 6 |
| 9 | 6 | 1 |
| 10 | 0 | 5 |
| 11 | 2 | 1 |

(c)an example of negative control: water

| Mood state | number of panelists in the mood state | |
|---|---|---|
| | Water | |
| depression | Before sniffing | after sniffing |
| 0 | 0 | 0 |
| 1 | 0 | 0 |
| 2 | 20 | 22 |
| 3 | 10 | 9 |
| 4 | 5 | 4 |
| 5 | 2 | 3 |
| 6 | 2 | 1 |
| 7 | 1 | 1 |
| 8 | 0 | 1 |
| 9 | 0 | 1 |
| 10 | 1 | 0 |
| 11 | 1 | 0 |

**Table 2**

| **Essential Oil** | **Reported Folklore Effects on Mood** |
|---|---|
| **Lavender** | Relaxing, anti-nervousness, reduces melancholy, relieves fatigue, |
| | anti-depressive, stimulating |
| | |
| **Rosemary** | Brain stimulant, invigorating, anti-depressive |
| | |
| **Rose** | Anti-stress, soothing, anti-depressive, enhances positive feelings |

### Example 3

The procedure described in Example I can be modified so as to include a nutrient medium on which the yeast can grow. An example of suitable media ingredients are as follows:

| **Ferment Media Ingredients** |
|---|
| Biospringer Yeast Extract 3g/L |
| Briess Malt Extract 3g/L |
| Marcor Pea Hydrolysate 5g/L |
| Glucose 10g/L |

The conversion process is conducted as described in the previous example, with the following modifications. The process will last from 24 - 72 hours depending on the rate of carbohydrate utilization. After the conversion is concluded, the final processing is done. No pyrolysis step is performed. The entire contents of the vessel are sonicated in glass beakers for 1 hour. Glass beads are added to the flask to help with the sonication process. After sonication, the medium is centrifuged in 200 ml vessels at 4000 RPM for 15 minutes. The centrifuged samples are then run through a 0.22 um filter. Once through the filter, the sample is preserved with 0.5% phenoxyethanol and set aside for further use.

### Example 4

The procedure of Example 3 is used to convert N-acetyl glucosamine and mannose-6-phosphate, each of which is known to have biological activity in the desquamation of skin cells. The effect of the bioconverted exfoliating agents is then tested for their ability to enhance exfoliation of the skin is evaluated, as follows.

### Study Design

The subjects included in this study were 120 females between the ages of 21 and 65 years, all meeting the screening criteria of good health and not being pregnant or lactating. The subjects reported for testing without moisturizers or any other products on their hands and baseline measurements were taken. They were randomly assigned to one of the following eight treatment groups. They were given the product to take home and self-administer to their right hand only, twice a day in the morning after washing and in the evening at least 15 minutes before bedtime for four weeks. The left hand served as the untreated control site. The subjects were only allowed to use the test product and specifically log its use in a daily diary provided. At the end of two and four weeks the subjects returned for testing without applying the product for at least 12 hours and they were re-evaluated under the same conditions.

### Skin exfoliation via D-Squame Discs Method and Image Analysis

Four D-Squame discs were firmly and evenly pressed on the face and the back of each hand with a hand held uniform pressure device and removed by gently pulling away from the skin. The D-Squame discs were mounted on clear microscope slides and labeled according to panelist name and visit. Desquamation was evaluated from the D-Squame discs via the image analyzer. Skin evaluation was carried out before treatment, and after two and four weeks of treatment.

The OPTIMA image analyzer was used to evaluate skin flakiness. The D-Squame samples containing the stratum comeocytes are placed under a camera on top of a light table and each image is imported into the image analyzer. The average Gray Value corresponding to the sample density is measured. The denser the sample the higher the Gray value difference.

### Test Products and Group Assignment:

The following test groups were assigned:
1. Placebo (D 1 base)
2. 1% Broth
3. Broth + yeast = 1% Ferment
4. 1% Broth + N-Acetyl Glucosamine
5. 1% Broth + Mannose 6-Phosphate
6. 1% Ferment of N-Acetyl Glucosamine
7. 1% Ferment of Mannose 6-Phosphate
8. 1 % Ferment of Mannose 6-Phosphate +Clary sage+Tourmaline

### Results

Skin exfoliation was evaluated by measuring the amount of flakes removed from the skin surface using D-Squame discs and analyzing them via the IA method. In this study several ferment combinations of N-Acetyl D-Glucosamine and Mannose 6-Phosphate vs. the placebo, the broth and the ferment as controls, for their effect on skin desquamation. The data clearly demonstrates that the fermentation process boosted the activity of both materials. The glucosamine consistently showed higher efficacy than the mannose 6-phosphate. The results are summarized in Table 3 below.

**Table 3**

| | **% Decrease in Flakiness** | | **p value** | |
|---|---|---|---|---|
| | **2 weeks** | **4 weeks** | **2 weeks** | **4 weeks** |
| **Placebo (D1 Base)** | **10.7** | **12.2** | **0.259** | **0.179** |
| Broth | 14.0 | 13.7 | 0.046 | 0.010 |
| *Broth* + *yeast* = *Ferment* | 13.2 | 17.1 | 0.029 | 0.031 |
| Broth + N-Acetyl Glucosamine | 24.5 | 30.7 | 0.000 | 0.000 |
| Broth + Mannose 6-Phosphate | 16.6 | 25.2 | 0.000 | 0.000 |
| Ferment of N-Acetyl Glucosamine | 27.7 | 36.4 | 0.000 | 0.000 |
| Ferment of Mannose 6-Phosphate | 22.2 | 28.2 | 0.000 | 0.000 |
| Ferment of Mannose 6-Phosphate + Clary sage+Tourmaline | 19.8 | 24.8 | 0.000 | 0.001 |

### Example 5

Another series of tests were conducted as described above. The concentration of N-acetyl D-glucosamine and mannose 6-phosphate was reduced by 10 fold without compromising their activity. Table 4 below summarizes the actual active concentrations used with a brief description along with their activity.

**Table 4**

| **Description** | **Concentration of active in formula** | **Decrease in Flakiness** | |
|---|---|---|---|
| | | **2 wk** | **4wk** |
| 1. Placebo (D1 base) | 0 | 11% | 12% |
| 2. 1% Broth (media containing nutrients for the yeast) | 0 | 14% | 14% |
| 3. 1% Ferment (Broth+yeast fermented 3-5 days) | 0 | 13% | 17% |
| 4. 1% (Broth containing 10% N-Acetyl Glucosamine) | 0.1% | 25% | 31% |
| 5. 1%(Broth containing 10% Mannose 6-Phosphate) | 0.1% | 17% | 25% |
| 6. 1 % (Ferment containing 10% N-Acetyl Glucosamine) | 0.1% | 28% | 36% |
| 7. 1% (Ferment containing 10% Mannose 6-Phosphate) | 0.1% | 22% | 18% |
| 8. 1% N-Acetyl Glucosamine ** | 1.0% | 16% | 27% |
| 9. 1% Mannose 6-Phosphate ** | 1.0% | 22% | 29% |

| | | | |
|---|---|---|---|
| ** Note: #'s 8&9 were tested previously in a base and the results shown represent their activity over the placebo. | | | |

## Claims

1. A method for manufacturing a bioconverted preparation, comprising the steps of:
a. incubating a plant or a non-human animal material selected for bioconversion in an aqueous environment for a period of time sufficient to extract active material from the plant or non-human animal material,
b. subjecting the material from (a) to a bioconversion process where the extract is combined with yeast microorganisms at room temperature, stirred at 20-200 rpm with bubbling air at 0.02-2 liters/minutes for a period of at least about 24 hours,
c. pyrolyzing by increasing the temperature to 90-95°C for a period of about 5-10 minutes,

2. The method of claim 1 in which the yeast is saccharomyces.

3. The method of claim 1 in which the microorganism is incubated with the extract in the substantial absence of any nutrient in the medium.

4. The method of claim 1 in which the microorganism exhibits substantially no growth during the incubation.

5. The method of claim 1 in which the extract is a crude plant extract.

6. The method of claim 1 in which the extract is a substantially pure plant-derived compound.

7. The method of claim 1 in which the extract is a crude non-human animal extract.

8. The method of claim 1 in which the extract is derived from the group consisting of glycyrrhia, matricaria, lavender, ginger, rose, juniper, spinach, green tea, white tea, white birch, pterocarpus, centella, aniba, echinacea, hypericum, neem, mimosa, rosemary, basil, grape and aloe.

9. The method of claim 1 in which the extract is derived from the group consisting of chitin, keratin, collagen, and cartilage.

10. The method of claim 1 in which the extract is a compound selected from the group consisting of flavonoids, isoflavonoids, anthocyanins, amino acids, aldosamines, xanthines, phosphosugars, resveratrol, rosmarinic acid, glycyrrhizin, steroids, and steroid precursors.

11. The method of claim 1 in which the extract is a plant essential oil.

12. The method of claim 1 in which the medium comprises at least one structured water.

13. The method of claim 1 in which the extract has been prepared in the presence of at least one structured water.

14. A bioconverted naturally occurring material prepared according to the method of claim 1.

15. The material of claim 14 which is derived from glycyrrhia, matricaria, lavender, ginger, rose, juniper, spinach, green tea, white tea, white birch, pterocarpus, centella, aniba, echinacea, hypericum, neem, mimosa, rosemary, basil, grape and aloe.

16. The material of claim 14 which is derived from chitin, keratin, collagen or cartilage.

17. The material of claim 14 which is derived from a compound selected from the group consisting of a compound selected from the group consisting of flavonoids, isoflavonoids, anthocyanins, amino acids, aldosamines, xanthines, phosphosugars, resveratrol, rosmarinic acid, glycyrrhizin, steroids and steroid precursors.

18. The method of claim 1 in which the medium is provided with sufficient nutrients for growth of the microorganism.

19. A bioconverted material prepared according to the method of claim 18.

20. The material of claim 19 which is derived from an aldosamine or a phosphosugar.

21. A cosmetic or pharmaceutical composition containing a biologically active amount of the bioconverter material of claim 14.

22. A cosmetic or pharmaceutical composition containing a biologically active amount of the bioconverted material of claim 19.

## Patentansprüche

1. Verfahren zur Herstellung eines biokonvertierten Präparats, das die Schritte umfasst:
a. Inkubieren eines pflanzlichen Materials oder eines Materials aus nicht-menschlichen Tieren, das zur Biokonversion ausgewählt wurde, in einer wässrigen Umgebung über einen Zeitraum, der ausreicht, um aktives Material aus dem pflanzlichen Material oder dem Material aus nicht-menschlichen Tieren zu extrahieren,
b. Unterwerfen des Materials aus (a) einem Biokonversionsprozess, wobei der Extrakt mit Hefemikroorganismen bei Raumtemperatur vereint und mit 0,02-2 Litern/Minute sprudelnder Luft von über einen Zeitraum von mindestens 24 Stunden mit 20-2000 UPM gerührt wird,
c. Pyrolysieren durch Erhöhen der Temperatur auf 90-95 °C über einen Zeitraum von etwa 5-10 Minuten.

2. Verfahren nach Anspruch 1, wobei die Hefe Saccharomyces ist.

3. Verfahren nach Anspruch 1, wobei der Mikroorganismus mit dem Extrakt im Wesentlichen ohne einen Nährstoff im Medium inkubiert wird.

4. Verfahren nach Anspruch 1, wobei der Mikroorganismus im Wesentlichen kein Wachstum während der Inkubation zeigt.

5. Verfahren nach Anspruch 1, wobei der Extrakt ein roher Pflanzenextrakt ist.

6. Verfahren nach Anspruch 1, wobei der Extrakt eine im Wesentlichen reine, aus Pflanzen stammende Verbindung ist.

7. Verfahren nach Anspruch 1, wobei der Extrakt ein Extrakt aus nicht-menschlichen Tieren ist.

8. Verfahren nach Anspruch 1, wobei der Extrakt aus der Gruppe stammt, die aus Glycyrrhia, Matricaria, Lavendel, Ingwer, Rose, Wacholder, Spinat, grünem Tee, weißem Tee, weißer Birke, Pterocarpus, Centella, Aniba, Echinacea, Hypericum, Neem, Mimose, Rosmarin, Basilikum, Traube und Aloe besteht.

9. Verfahren nach Anspruch 1, wobei der Extrakt aus der Gruppe stammt, die aus Chitin, Keratin, Kollagen und Knorpel besteht.

10. Verfahren nach Anspruch 1, wobei der Extrakt eine Verbindung ist, die ausgewählt ist aus der Gruppe bestehend aus Flavonoiden, Isoflavonoiden, Anthocyanen, Aminosäuren, Aldosaminen, Xanthinen, Phosphozuckern, Resveratrol, Rosmarinsäure, Glycyrrhizin, Steroiden und Steroidvorläufern.

11. Verfahren nach Anspruch 1, wobei der Extrakt ein essentielles Pflanzenöl ist.

12. Verfahren nach Anspruch 1, wobei das Medium mindestens ein strukturiertes Wasser umfasst.

13. Verfahren nach Anspruch 1, wobei der Extrakt in Gegenwart von mindestens einem strukturierten Wasser hergestellt wurde.

14. Biokonvertiertes natürlicherweise vorkommendes Material, das gemäß dem Verfahren nach Anspruch 1 hergestellt wird.

15. Material nach Anspruch 14, das aus Glycyrrhia, Matricaria, Lavendel, Ingwer, Rose, Wacholder, Spinat, grünem Tee, weißem Tee, weißer Birke, Pterocarpus, Centella, Aniba, Echinacea, Hypericum, Neem, Mimose, Rosmarin, Basilikum, Traube und Aloe stammt.

16. Material nach Anspruch 14, das aus Chitin, Keratin, Kollagen oder Knorpel stammt.

17. Material nach Anspruch 14, das von einer Verbindung abgeleitet ist, die ausgewählt ist aus der Gruppe bestehend aus Flavonoiden, Isoflavonoiden, Anthocyanen, Aminosäuren, Aldosaminen, Xanthinen, Phosphozuckern, Resveratrol, Rosmarinsäure, Glycyrrhizin, Steroiden und Steroidvorläufern.

18. Verfahren nach Anspruch 1, wobei das Medium mit genügend Nährstoffen zum Wachstum des Mikroorganismus versehen ist.

19. Biokonvertiertes Material, das gemäß dem Verfahren nach Anspruch 18 hergestellt wird.

20. Material nach Anspruch 19, das von einem Aldosamin oder einem Phosphozucker abgeleitet ist.

21. Kosmetische oder pharmazeutische Zusammensetzung, die eine biologisch aktive Menge des biokonvertierten Materials von Anspruch 14 enthält.

22. Kosmetische oder pharmazeutische Zusammensetzung, die eine biologisch aktive Menge des biokonvertierten Materials von Anspruch 19 enthält.

## Revendications

1. Procédé pour la fabrication d'une préparation bioconvertie, comprenant les étapes consistant à :
a. incuber un matériau végétal ou animal non humain choisi pour la bioconversion dans un environnement aqueux pendant une période de temps suffisante pour extraire le matériau actif du matériau végétal ou animal non humain,
b. soumettre le matériau obtenu en (a) à un procédé de bioconversion où l'extrait est combiné avec des microorganismes de type levure à température ambiante, agité à 20 à 200 t/min avec barbotage d'air à 0,02 à 2 litres/minute pendant une période d'au moins environ 24 heures,
c. pyrolyser par élévation de la température à 90 à 95°C pendant une période d'environ 5 à 10 minutes.

2. Procédé selon la revendication 1, dans lequel la levure est saccharomyces.

3. Procédé selon la revendication 1, dans lequel le microorganisme est incubé avec l'extrait pratiquement en l'absence d'un quelconque nutriment dans le milieu.

4. Procédé selon la revendication 1, dans lequel le microorganisme ne présente pratiquement pas de croissance durant l'incubation.

5. Procédé selon la revendication 1, dans lequel l'extrait est un extrait végétal brut.

6. Procédé selon la revendication 1, dans lequel l'extrait est un composé d'origine végétale pratiquement pur.

7. Procédé selon la revendication 1, dans lequel l'extrait est un extrait d'animal non humain brut.

8. Procédé selon la revendication 1, dans lequel l'extrait est dérivé du groupe constitué par glycyrrhia, matricaria, la lavande, le gingembre, la rose, la genièvre, l'épinard, le thé vert, le thé blanc, le bouleau blanc, pterocarpus, centella, aniba, l'échinacée, hypericum, le neem, le mimosa, le romarin, le basilic, le raisin et l'aloès.

9. Procédé selon la revendication 1, dans lequel l'extrait est dérivé du groupe constitué par la chitine, la kératine, le collagène, et le cartilage.

10. Procédé selon la revendication 1, dans lequel l'extrait est un composé choisi dans le groupe constitué par les flavonoïdes, les isoflavonoïdes, les anthocyanines, les acides aminés, les aldosamines, les xanthines, les phosphosucres, le resvératrol, l'acide rosmarinique, la glycyrrhizine, les stéroïdes, et les précurseurs de stéroïdes.

11. Procédé selon la revendication 1, dans lequel l'extrait est une huile essentielle de plante.

12. Procédé selon la revendication 1, dans lequel le milieu comprend au moins une eau structurée.

13. Procédé selon la revendication 1, dans lequel l'extrait a été préparé en présence d'au moins une eau structurée.

14. Matériau naturel bioconverti préparé conformément au procédé de la revendication 1.

15. Matériau selon la revendication 14, qui est dérivé de glycyrrhia, matricaria, lavande, gingembre, rose, genièvre, épinard, thé vert, thé blanc, bouleau blanc, pterocarpus, centella, aniba, échinacée, hypericum, neem, mimosa, romarin, basilic, raisin et aloès.

16. Matériau selon la revendication 14, qui est dérivé de chitine, kératine, collagène ou cartilage.

17. Matériau selon la revendication 14, qui est dérivé d'un composé choisi dans le groupe constitué par les flavonoïdes, les isoflavonoïdes, les anthocyanines, les acides aminés, les aldosamines, les xanthines, les phosphosucres, le resvératrol, l'acide rosmarinique, la glycyrrhizine, les stéroïdes, et les précurseurs de stéroïdes.

18. Procédé selon la revendication 1, dans lequel le milieu est pourvu de suffisamment de nutriments pour la croissance du microorganisme.

19. Matériau bioconverti préparé conformément au procédé de la revendication 18.

20. Matériau selon la revendication 19, qui est dérivé d'une aldosamine ou d'un phosphosucre.

21. Composition cosmétique ou pharmaceutique contenant une quantité biologiquement active du matériau bioconverti de la revendication 14.

22. Composition cosmétique ou pharmaceutique contenant une quantité biologiquement active du matériau bioconverti de la revendication 19.
